# EUROPÄISCHE PATENTSCHRIFT

(11) **EP 3 999 124 B1**
(45) Veröffentlichungstag und Bekanntmachung des Hinweises auf die Patenterteilung: **02.09.2026**
(21) Anmeldenummer: 19749257.2
(22) Anmeldetag: 19.07.2019
(51) Int. Cl.: A61L 2/10, A61L 9/20

(54) **VERFAHREN ZUR INAKTIVIERUNG VON PATHOGENEN MIKROORGANISMEN UND VIREN IN EINEM EINZEL- ODER GRUPPENSTALL FÜR JUNGTIERE**
METHOD FOR INACTIVATING PATHOGENIC MICROORGANISMS AND VIRUSES IN AN INDIVIDUAL OR GROUP HOUSING FOR YOUNG ANIMALS
DISPOSITIF ET PROCÉDÉ D'INACTIVATION DE MICROORGANISMES ET DE VIRUS PATHOGÈNES DANS UNE ÉTABLE INDIVIDUELLE OU DE GROUPE POUR DES ANIMAUX JUVÉNILES

(43) Veröffentlichungstag der Anmeldung: 25.05.2022
(73) Patentinhaber: Urban Holding GmbH, 27798 Hude/Wüsting (DE)
(72) Erfinder: SPROCK, Thomas, 27798 Hude-Wüsting (DE)
(74) Vertreter: Manasse, Uwe
(86) Internationale Anmeldenummer: PCT/EP2019/069579
(87) Internationale Veröffentlichungsnummer: WO 2021/013323

(56) Entgegenhaltungen:
- WO-A1-2013/116566
- WO-A1-2016/164362
- CN-Y- 2 281 473
- US-A1- 2017 290 935
- US-B2- 6 979 103
- US-B2- 7 459 694
- US-B2- 8 748 829

## Beschreibung

Die vorliegende Erfindung betrifft ein Verfahren zur Inaktivierung von pathogenen Mikroorganismen in einem Einzel- oder Gruppenstall für Jungtiere, insbesondere für landwirtschaftlichen Nutztiere, insbesondere Kälber.

Die vorliegende Erfindung betrifft das technische Gebiet der landwirtschaftlichen Nutztierhaltung, speziell die Haltung von Jungtieren.

Beispielsweise bei der landwirtschaftlichen Milchviehhaltung werden neugeborene Kälber zumindest in den ersten Lebenstagen zumeist in Einzelhaltung aufgezogen. Typische hierfür verwendete Einzelbehausungen (Einzelstall) sind Kälberiglus oder Kälberhütten. Im weiteren Verlauf der Aufzucht werden die Kälber dann meist in Gruppen (in Gruppenställen) aufgestallt.

Bei neugeborenen Kälbern handelt es sich um empfindliche Jungtiere, die ohne aktiven Immunschutz geboren werden und demgemäß anfällig gegen Krankheiten sind.

Bei den Kälberkrankheiten herrschen insbesondere Atemwegs- und Durchfallerkrankungen vor. Beide Erkrankungen sind infektiös und können daher schnell einen Großteil der Tiergruppe betreffen.

Durchfallerkrankungen können fütterungs- und hygienebedingte Ursachen haben, oftmals ist die Ursache der Erkrankung eine Infektion mit Krankheitserregern.

Häufig anzutreffende, Durchfall verursachende Krankheitserreger bei Kälbern sind zum einen Viren, wie etwa Rota- und/oder Coronaviren. Als weitere Erreger von Kälberdurchfällen sind Kryptosporidien, eine Gattung einzelliger Parasiten, zu benennen. Speziell die Spezies Cryptosporidium parvum verursacht Durchfälle bei Kälbern, sehr oft bereits in den ersten beiden Lebenswochen. Von Durchfall betroffene Kälber weisen massive gesundheitliche Probleme auf, in deren Folge es zu erheblichen wirtschaftlichen Schäden für den Tierhalter kommt.

Kryptosporidien kommen, wie sehr viele andere Endoparasiten auch, in verschiedenen Entwicklungsstadien vor und haben einen komplexen Lebenszyklus.

Kryptosporidien bilden als Dauerstadien sehr widerstandsfähige Oocysten aus, die hochinfektiös sind und dies über Monate hinweg bleiben. In einem Gramm Kot eines infizierten Tieres können sich bis zu 5 Millionen Oocysten befinden.

Bereits die orale Aufnahme von weniger als 100 Oocysten kann zur Infektion eines Kalbes führen. Nach einer erfolgten Infektion vermehren sich die Kryptosporidien im Verdauungstrakt des Tieres sehr schnell und effektiv. Die gebildeten Oocysten werden dann wieder mit dem Kot ausgeschieden und können weitere Tiere infizieren.

Die Behandlung einer Kryptosporidiose ist teuer und ineffektiv. Es gibt kaum Medikamente zur Bekämpfung dieser Krankheit und die einzigen vorliegenden Arzneimittel sind mit starken Nebenwirkungen verbunden. Insofern ist und bleibt das wirksamste Mittel zur Verhinderung und/oder Bekämpfung der Krankheit das Schaffen und Aufrechterhalten einer hygienischen Haltungsumgebung.

Cryptosporidium-Oocysten verfügen über eine sehr stabile Hülle. Sie sind sehr langlebig und können Wochen und Monate überdauern. Mittels einer herkömmlichen Stallreinigung (bloßes Ausspritzen der Abteile mit einem Hochdruckreiniger) sind Kryptosporidien nicht wirksam zu bekämpfen. Der Einsatz von Desinfektionsmitteln ist obligat für eine wirksame Bekämpfung und Eindämmung von durch Kryptosporidien verursachten Neugeboreneninfektionen.

Desinfektionsmittel sind jedoch vergleichsweise teure Verbrauchsmittel, die immer nur spezifisch gegen einen oder mehrere bestimmte Erreger wirksam sind. Ein universelles, alle Keime und Bakterien abtötendes Breitband-Desinfektionsmittel gibt es nicht. Da die Oocysten aufgrund ihrer sehr stabilen Hülle unempfindlich gegen herkömmliche Desinfektionsmittel auf Chlorid-Basis sind, müssen sie mit speziellen Desinfektionsmitteln behandelt werden. Diese Behandlung ist teuer und zeitaufwändig, erfordert sie doch mehrere Schritte: Grobreinigung des Stalls mit einem Hochdruckreiniger, Ausbringen und Einwirken des Desinfektionsmittelns, Ausspritzen und anschließendes Austrocknen des Stalls.

Ferner ist der Einsatz von Desinfektionsmitteln insofern problematisch, als dass es sich bei den eingesetzten Stoffen durchweg um Biozide handelt, die potentiell sowohl für den Anwender als auch für die Umwelt schädlich sind, was entsprechende Schutzmaßnahmen erforderlich macht, die auf landwirtschaftlichen Betrieben nicht immer eingehalten werden können. Zudem besteht die Gefahr, dass die zu bekämpfenden Mikroorganismen früher oder später Resistenzen gegen die Wirkstoffe der Desinfektionsmittel ausbilden, welche deren Wirksamkeit einschränken oder diese im Lauf der Zeit gänzlich unwirksam werden lassen.

Die Problematik der Akkumulation von pathogenen Mikroorganismen in Tierställen ist wohlbekannt, insbesondere bei größeren Stalleinheiten. Gängige Gegenmaßnahmen sind die Anwendung des sog. Rein-Raus-Verfahrens bei der Belegung der Stalleinheiten und die routinemäßige Reinigung der Abteile vor der Neubelegung, bei Bedarf zusätzlich flankierend begleitet von der Desinfektion derselben.

Die EP1138196 offenbart eine Tierunterkunft samt zugehörigem Zaun, geeignet zur Aufstallung von Jungtieren, speziell Kälbern, in Einzelhaltung. Wie aus der landwirtschaftlichen Praxis bekannt ist, kann durch die Aufstallung in Einzelhaltung der Keimdruck, welchem das Einzeltier ausgesetzt ist, wesentlich reduziert werden und die Tiergesundheit verbessert werden.

Noch einen Schritt weiter geht die DE202014006602, sie offenbart die Auskleidung einer solchen Kälberbox oder eines solchen Kälberiglus mit einer Folie, welche die Elemente bedeckt, die mit dem Kalb in Berührung kommen. Nach der Ausstallung eines Kalbes wird die Folie samt aufliegendem Stroh, Kot und Harn entsorgt und das nächste Kalb kann sofort in eine saubere und keimarme Stallumgebung ein- gestallt werden.

Die US 7 459 694 B2 offenbart eine Vorrichtung zur Inaktivierung von pathogenen Mikroorganismen, umfassend eine UV-C-Strahlenquelle zu besagter Inaktivierung, die auf einem Stativ befestigt ist. Die CN 2 281 473 Y, WO 2013/116566 A1, US 8 748 829 B2, US 6 979 103 B2, WO 2016/164362 A1 und US 2017/290935 A1 offenbaren ähnliche Vorrichtungen.

Der vorliegenden Erfindung liegt nun die Aufgabe zugrunde, pathogene Mikroorganismen in der Stallumgebung von Jungtieren, insbesondere von vornehmlich jungen landwirtschaftlichen Nutztieren, wie z.B. Kälbern, stark zu inaktivieren.

Erfindungsgemäß wird diese Aufgabe gelöst durch
ein Verfahren zur Inaktivierung von pathogenen Mikroorganismen in einem Einzel- oder Gruppenstall für Jungtiere, insbesondere für landwirtschaftlich Nutztiere, wie z.B. Kälber, gemäß Anspruch 1.

Bei dem Verfahren kann vorgesehen sein, dass es ferner Desinfektion des Einzel- oder Gruppenstalls vor der Bestrahlung mit UV-C-Strahlung umfasst.

Der vorliegenden Erfindung liegt die überraschende Erkenntnis zugrunde, dass, indem insbesondere exponierte Elemente der Stallumgebung, die von Mikroorganismen besiedelt sind, UV-C-Strahlung ausgesetzt werden, eine stark keimreduzierende Wirkung in Einzel- oder Gruppenställen für Jungtiere, insbesondere für landwirtschaftliche Nutztiere, z.B. Kälber, erzielt werden kann.

Die Erfindung setzt hierzu nicht auf den problembehafteten Einsatz von Desinfektionsmitteln, sondern nutzt UV-C-Strahlung zur Inaktivierung der pathogenen Mikroorganismen in der Stallumgebung. Eine Inaktivierung kann sowohl eine Abtötung als auch eine Verhinderung einer Reduplikation der DNA umfassen und eine Reduzierung der pathogenen Mikroorganismen bedeuten.

Kryptosporidien-Oozysten sind sehr empfindlich gegen UV-C Strahlung. In Trinkwasser auftretende Oocysten können mittels der Bestrahlung mit ultravioletter Strahlung mit einer Wellenlänge von 200 bis 300 nm und einer Dosis von 1 bis 175 Millijoule pro Quadratzentimeter inaktiviert werden.

UV-C-Strahlung ist jedoch nicht nur bei der Bekämpfung von Kryptosporidien-Oozysten wirksam, sondern kann gleichermaßen auch für die Inaktivierung von anderen Bakterien, Viren, Keimen und Mikroorganismen eingesetzt werden.

Bei UV-Licht handelt es sich um elektromagnetische Strahlung mit einer Wellenlänge im Bereich von 100 nm bis 390 nm. Die Wellenlänge dieses Licht liegt außerhalb des vom menschlichen Auge sichtbaren Spektralbereichs, kann aber etwa von Bienen durchaus wahrgenommen werden.

UV-Licht selbst wird in Abhängigkeit von der Wellenlänge wiederum in drei Kategorien eingeteilt: UV-A-Strahlung (Wellenlänge von 380 nm bis 315 nm), UV-B-Strahlung (315 nm bis 280 nm) und UV-C-Strahlung (280 nm bis 100 nm).

Die von der Sonne emittierte UV-A- und UV-B-Strahlungen dringen beide auf die Erde durch. Insbesondere UV-B-Strahlung wird von Mensch und Tier für die Vitamin D3-Bildung benötigt. Bei übermäßiger Exposition besteht jedoch die Gefahr von Sonnenbrand. Bei beständiger, langanhaltender Exposition steigt das Risiko an, an Hautkrebs zu erkranken.

Demgegenüber wird die UV-C-Strahlung von den obersten Luftschichten der Erdatmosphäre absorbiert, selbst im Bereich des Ozonlochs.

Die UV-C-Strahlung ist die energiereichste der drei UV-Strahlungstypen. Sie dringt zwar kaum in die Haut ein, schädigt jedoch massiv das Erbmaterial und wirkt daher stark keimreduzierend.

Die massive Schädigung des Erbmaterials (DNA) durch UV-C-Licht beruht darauf, dass die Photonen der UV-C-Strahlung eine Brückenbildung von zwei nebeneinander auf einem DNA-Helixstrang liegenden Thymin-Basen bewirken können. Durch die Ausbildung dieser sog. Thymindimere wird die Replikation und Transkription der DNA effektiv unterbunden. Eine durch UV-C-Bestrahlung derart veränderte Zelle kann sich nicht mehr teilen und keine neuen Proteine mehr herstellen. Sie stirbt ab. Die vorliegende Erfindung setzt zum Zweck der Keimreduzierung gänzlich oder in Kombination mit anderen Maßnahmen auf den Einsatz von UV-C-Strahlung gemäß dem oben beschriebenen Wirkmechanismus.

Die höchste Wirksamkeit bezüglich der Keimreduzierung wird bei Bestrahlung mit UV-Licht in einem Spektralbereich von 250 nm bis 270 nm erzielt, da in diesem Wellenbereich die Absorption der Strahlung durch die DNA am höchsten ist. Insofern sind für die Keimreduzierung vorzugsweise spezifische UV-C-Strahler zu verwenden, deren Emissionscharakteristik auf diese Anforderungen hin angepasst ist.

Im Vergleich zum bereits beschriebenen Alternativverfahren der Keimreduzierung mittels Desinfektionsmitteln weist der Einsatz von UV-C-Strahlung zu diesem Zweck wesentliche Vorteile auf: die durch ein Photon ausgelöste Bildung eines Thymindimers geht innerhalb von Sekundenbruchteilen vonstatten. Zwar kann ggf. eine DNA-Reparatur der geschädigten Stelle des Genmaterials stattfinden (sog. Photoreaktivierung oder Exzisionsreparatur, auch eine Dunkelreaktivierung ist möglich), jedoch können im Gegensatz zum Einsatz von Desinfektionsmitteln bzw. Antibiotika keinerlei Resistenzen gegen UV-Licht gebildet werden. Das Verfahren bleibt dauerhaft wirksam.

Die Nutzung der UV-C-Strahlung zur Keimreduzierung ist eine sehr energieeffiziente und damit kostengünstige Möglichkeit der Desinfektion, welches insbesondere auf glatten, sauberen Oberflächen sehr gut wirkt, wobei ein direkter Zusammenhang zwischen der Strahlendosis und der letalen Wirkung der Strahlung zu verzeichnen ist.

Ein weiterer Vorteil des Einsatzes von UV-C-Strahlung zur Keimreduzierung besteht darin, dass die eigentliche Desinfektionswirkung augenblicklich eintritt. Für eine ausreichende Keimreduzierung sind vergleichsweise kurze Bestrahlungsdauern erforderlich. Das Verfahren zur Keimreduzierung geht hinlänglich schnell vonstatten, ist energiesparend und chemikalienfrei und damit umweltschonend.

Wie bereits erwähnt, ist die UV-C-Strahlung auch gegen Dauerstadien von Mikroorganismen sehr gut wirksam ist. Somit eignen sich UV-C-basierte Verfahren sehr gut zur Bekämpfung von Kryptosporidien, welche in der Kälberhaltung oft ernsthafte Probleme verursachen, da die Oozysten (= Dauerstadien der Kryptosporidien) mit herkömmlichen Desinfektionsmitteln auf Chlorid-Basis nicht zu bekämpfen sind. Insofern kann durch den gezielten Einsatz von UV-Strahlung den gefürchteten und extrem schlecht therapierbaren Kryptosporidiosen vorgebeugt werden.

Weitere Merkmale und Vorteile der Erfindung ergeben sich aus den beigefügten Ansprüchen und aus der nachfolgenden Beschreibung, in der mehrere Ausführungsbeispiele anhand von schematischen Zeichnungen im Einzelnen erläutert werden. Dabei zeigt:
- Fig. 1: eine Vorrichtung zur Inaktivierung von pathogenen Mikroorganismen in einem Einzel- oder Gruppenstall für Jungtiere gemäß einer nicht beanspruchten Ausführungsform;
- Fig. 2: die Vorrichtung von Fig. 1 beim Einsatz zur Inaktivierung von auf einer Bodenfläche eines Einzel- oder Gruppenstalls befindlichen pathogenen Mikroorganismen;
- Fig. 3: eine Vorrichtung zur Inaktivierung von pathogenen Mikroorganismen in einem Einzel- oder Gruppenstall für Jungtiere gemäß einer weiteren nicht beanspruchten Ausführungsform;
- Fig. 4: die Vorrichtung von Fig. 1 noch einmal im Einsatz bei der Inaktivierung von auf einer Bodenfläche eines Einzel- oder Gruppenstalls befindlichen pathogenen Mikroorganismen;
- Fig. 5: eine Vorrichtung zur Inaktivierung von pathogenen Mikroorganismen in einem Einzel- oder Gruppenstall für Jungtiere gemäß einer weiteren nicht beanspruchten Ausführungsform; und
- Fig. 6: eine Vorrichtung zur Inaktivierung von pathogenen Mikroorganismen in einem Einzel- oder Gruppenstall für Jungtiere gemäß einer weiteren nicht beanspruchtenAusführungsform.

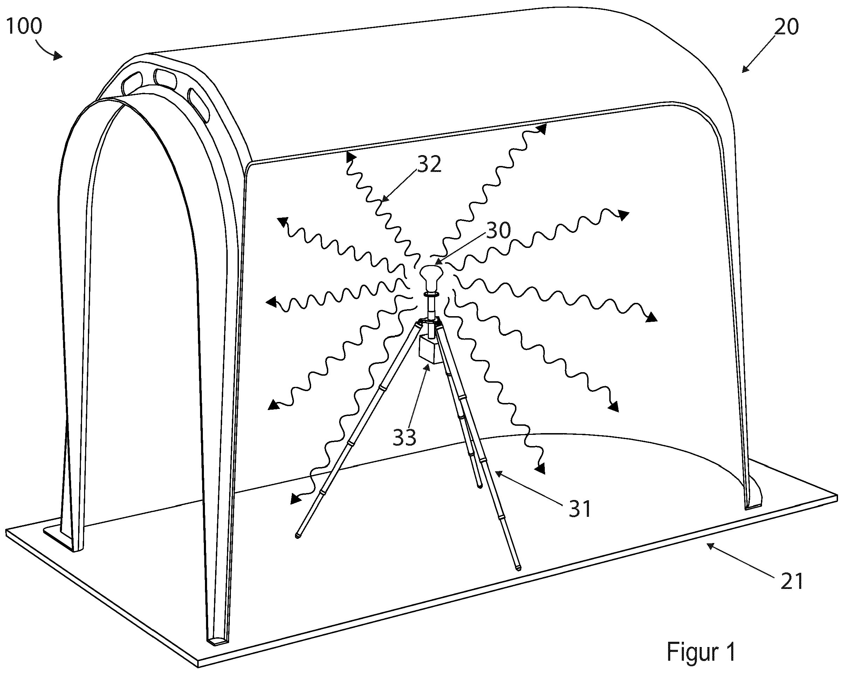

FIG. 1 zeigt eine Vorrichtung 100 zur Inaktivierung von pathogenen Mikroorganismen in einem Einzel- oder Gruppenstall für Jungtiere, insbesondere für landwirtschaftliche Nutztiere, z.B. Kälber mit UV-C Strahlung 32: innerhalb eines Einzelstalls 20, vorzugsweise inmitten desselben ist eine UV-C Strahlenquelle 30 montiert oder aufgehängt, welche UV-C-Strahlung 32 auf die Innenwände und die Bodenfläche 21 des Einzelstalls aussendet, um das Erbgut von auf den Innenwänden oder der Bodenfläche angesiedelten Krankheitserregern (Bakterien und/oder Keimen) zu schädigen und diese damit zum Absterben zu bringen. Vorzugsweise wird die Bestrahlung nach einer erfolgten Reinigung des nicht belegten Einzelstalls durchgeführt. Die UV-C-Strahlenquelle 30, deren Spannungsversorgung idealerweise über einen Akku bzw. über eine Batterie erfolgt, ist in diesem Beispiel auf einem Stativ 31 montiert oder kann z.B. von der Decke des Einzelstalls 20 abgehängt werden. Die Bestrahlung wird nach Start der UV-C-Strahlenquelle 30 automatisiert für eine bestimmte Dauer fortgesetzt. Aus Gründen der Arbeitssicherheit und des Strahlenschutzes sind vorzugsweise im Einzelstall Zugänge zu verkleiden bzw. so abzuschirmen, dass Arbeitspersonen nicht unwissentlich oder versehentlich der schädigenden UV-C-Strahlung ausgesetzt sein können.

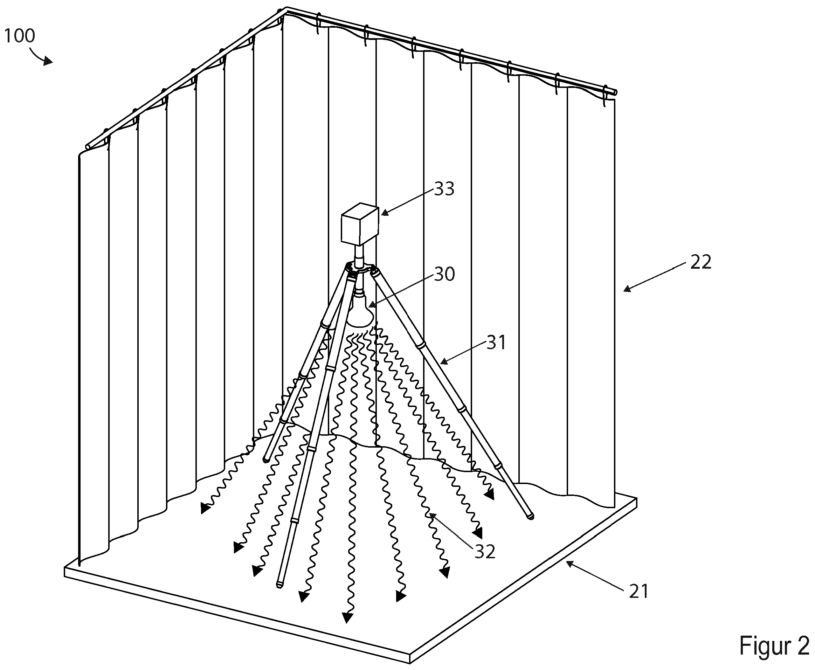

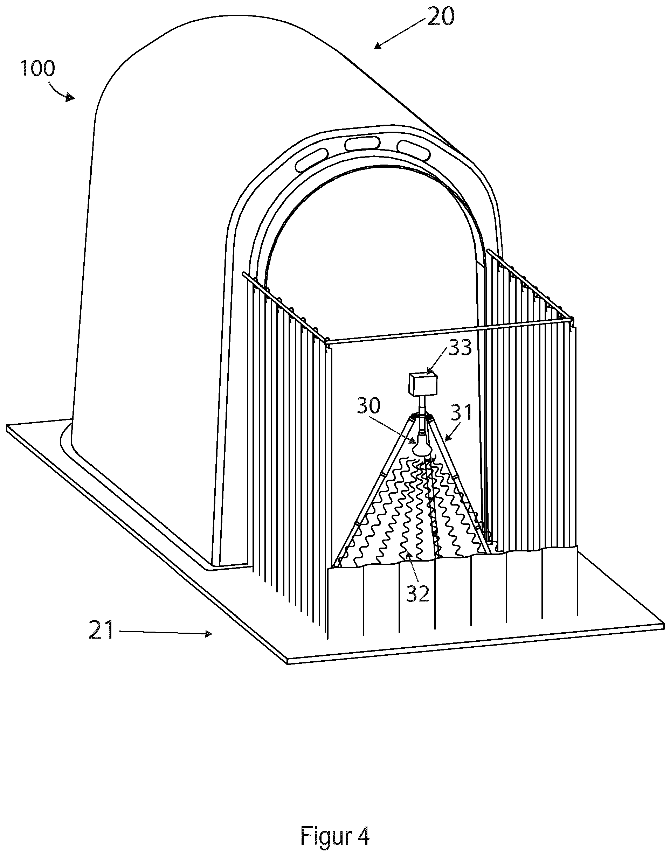

FIG. 2 sowie FIG. 4 zeigen die Vorrichtung 100 von Fig. 1 eingesetzt zur Inaktivierung von auf der Bodenfläche 21 von Einzel- und/oder Gruppenställen befindlichen Krankheitserregern. Vorzugsweise wird auch hier die Bestrahlung nach einer erfolgten Reinigung der Bodenfläche durchgeführt, wobei die UV-C-Strahlenquelle 30 automatisiert nach Ablauf einer vorgebbaren bestimmten Zeitdauer abgeschaltet wird. Eine Verkleidung bzw. Abschirmung der UV-C Strahlenquelle kann z.B. über einen Vorhang 22 oder z.B. eine zeltartige Kuppel (nicht gekennzeichnet) erfolgen. Die Vorrichtung ist gleichermaßen für die Bodenflächen eines Einzelstalls (FIG. 4) wie auch für Bodenflächen in Gruppenställen oder anderen Stalltypen (FIG. 2) einsetzbar.

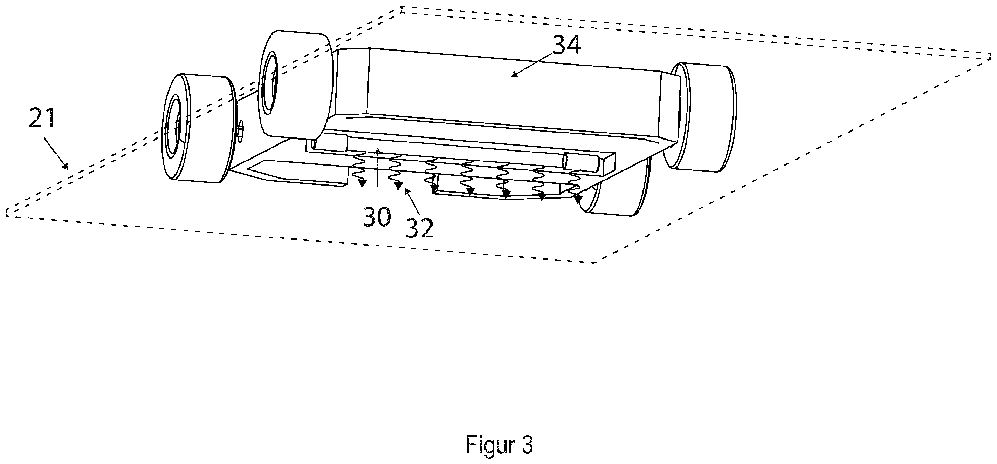

FIG. 3 zeigt eine weitere Ausführungsform der Vorrichtung 100 eingesetzt zur

Inaktivierung von auf der Bodenfläche 21 von Stallungen befindlichen Krankheitserregern. Hierbei ist die UV-C-Strahlenquelle 30 auf der Unterseite einer autonom fahrfähig bzw. fahrenden Plattform 34 angebracht, welche selbständig auf Lauf- und/oder Liegeflächen eines Einzel- oder Gruppenstalls manövriert. Zielsetzung ist es in diesem Beispiel, alle Bereiche der Bodenfläche ein oder mehrmalig zu überfahren und in diesem Zuge die gesamte Bodenfläche mit keimreduzierender UV-C Strahlung 32 zu behandeln.

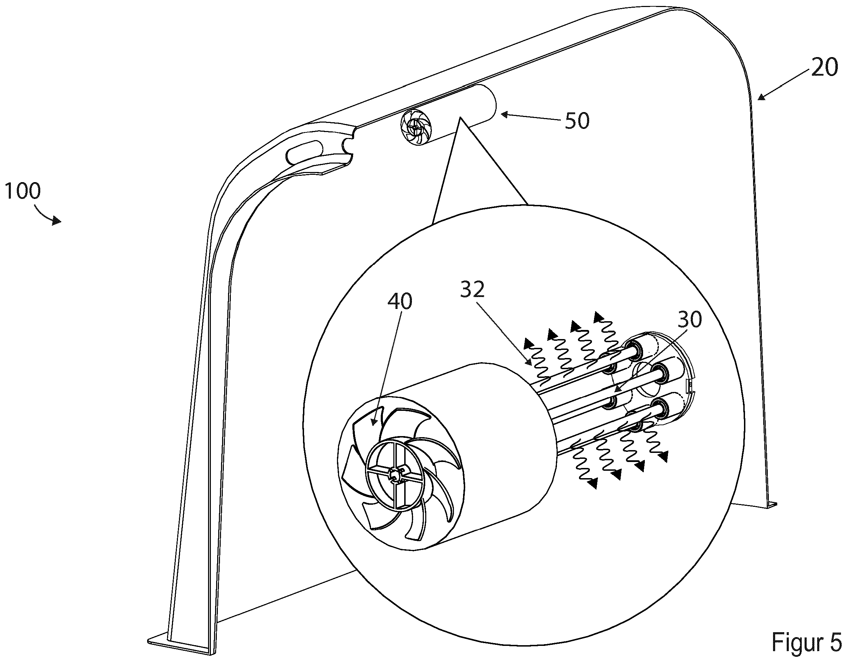

FIG. 5 zeigt eine weitere Ausführungsform der Vorrichtung 100 zur Inaktivierung von (in der Stallluft befindlichen) pathogenen Mikroorganismen mittels UV-C Strahlung 32: hierbei ist die UV-C-Strahlenquelle 30 innerhalb eines Luftumwälzelements 50 platziert, welches mittels eines Ventilators 40 die Luft innerhalb eines Einzel- oder Gruppenstalls 20 für landwirtschaftliche Nutztiere umwälzt. Von der UV-C-Strahlenquelle 30 ausgesendete UV-Strahlung 32 trifft auf die in der Umluft befindlichen, über die Luftumwälzung herbei transportierten pathogenen Mikroorganismen und inaktiviert diese. Vorzugsweise sollte die emittierende Oberfläche der UV-C-Strahlenquelle 30 vorzugsweise periodisch von Stallstaub befreit werden, um deren Wirksamkeit zu erhalten. Idealerweise erfolgt das Freiblasen der UV-C-Strahlenquelle mittels des Ventilators (Umwälzventilators) 40.

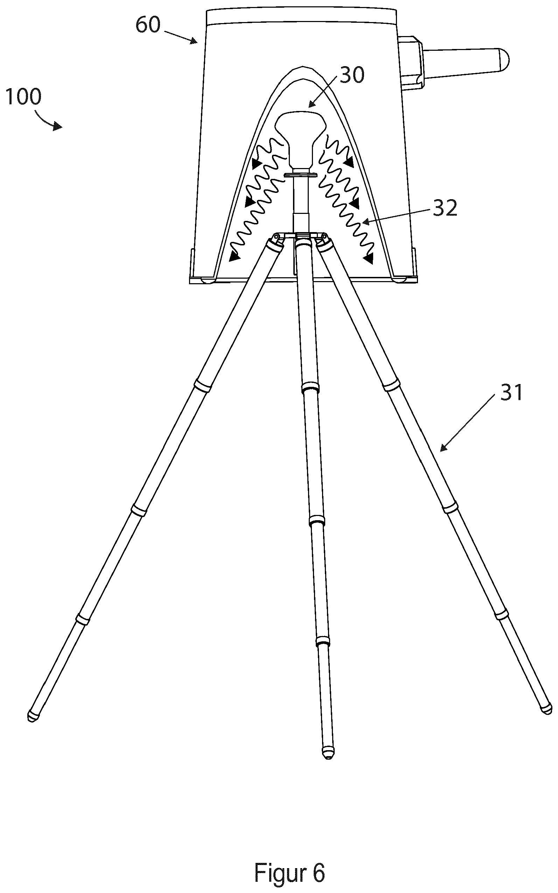

FIG. 6 zeigt die Vorrichtung 100 von Figur 1 bzw. gemäß einer weiteren besonderen Ausführungsform eingesetzt zur Inaktivierung von Krankheitserregern, die sich auf der inneren Oberfläche eines Anmischbehälters (Tränke- oder Futtereimers) 60 befinden. In diesem Beispiel umfasst die Vorrichtung 100 einen Halter (nicht gezeigt) für den Anmischbehälter 60 und es wird die UV-C-Strahlenquelle 30 von unten in den im Halter mit der Öffnung nach unten gehaltenen Anmischbehälter 60 eingeführt und im Inneren des Anmischbehälters 60 positioniert. Vorzugsweise wird auch hier die Bestrahlung nach einer erfolgten Reinigung des Anmischbehälters durchgeführt, wobei vorzugsweise die UV-C-Strahlenquelle 30 automatisiert nach Ablauf einer bestimmten Zeitdauer abgeschaltet wird. Da die UV-C-Strahlenquelle durch den Anmischbehälter 60 abgeschirmt wird, kann auf eine weitere Vorrichtung zum Strahlenschutz verzichtet werden.

In der obigen Beschreibung wurden drei Maßnahmen zur Inaktivierung von pathogenen Mikroorganismen dargelegt. Es handelt sich dabei um:
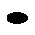 Bestrahlung einer Einzel- oder Gruppenbehausung (Einzel- oder Gruppenstall) für landwirtschaftliche Nutztiere mit UV-C-Strahlung zum Zwecke der Abtötung von auf den Innenwänden der Behausung angesiedelten pathogenen Mikroorganismen,
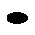 Bestrahlung der Bodenfläche einer Stallung für Tiere mit UV-C-Strahlung zum Zwecke der Abtötung von auf der Bodenfläche befindlichen pathogenen Mikroorganismen, ggf. kann dabei die Bestrahlung von einem autonom fahrenden Keimreduzierungsroboter vorgenommen werden,
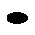 Umwälzung und Bestrahlung der Luft innerhalb einer Einzel- oder Gruppenbehausung für landwirtschaftliche Nutztiere mit UV-C-Strahlung zum Zwecke der Abtötung von in der Luft befindlichen pathogenen Mikroorganismen, und
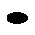 Bestrahlung eines Tränke- oder Futtereimers für landwirtschaftliche Nutztiere mit UV-C-Strahlung zum Zwecke der Abtötung von auf den Innenwänden des Eimers angesiedelten pathogenen Mikroorganismen.

Diese Maßnahmen können einzeln oder in beliebigen Kombinationen erfolgen.

## Patentansprüche

1. Verfahren zur Inaktivierung von pathogenen Mikroorganismen in einem Einzel- oder Gruppenstall (20) für Jungtiere, umfassend eine UV-C-Strahlenquelle (30) zu besagter Inaktivierung, die auf einem Stativ (31) in einem Einzel- oder Gruppenstall (20) angeordnet oder von einer Decke in einem Einzel- oder Gruppenstall (20) abgehängt oder an der Decke befestigt ist, umfassend die Schritte:
- Ausstallung eines Jungtieres in einem Einzelstall (20) oder von Jungtieren in einem Gruppenstall (20),
- Entmisten des Einzel- oder Gruppenstalls (20),
- Reinigung des Einzel- oder Gruppenstalls (20), und
- Bestrahlung der Bodenfläche (21), vorzugweise der gesamten inneren Oberfläche, des Einzel- oder Gruppenstalls (20) mit UV-C-Strahlung (32).

2. Verfahren nach Anspruch 1, ferner umfassend Desinfektion des Einzel- oder Gruppenstalls (20) vor der Bestrahlung mit UV-C-Strahlung (32).

3. Verfahren nach Anspruch 1 oder 2, wobei bei Verwendung besagter Vorrichtung (100) diese ferner einen Halter für einen Anmischbehälter (60) umfasst, wobei der Halter so konfiguriert ist, dass die UV-C-Strahlenquelle (30) aktiv oder passiv im Inneren eines mittels des Halters gehaltenen Anmischbehälters (60) positionierbar ist.

## Claims

1. Method for inactivating pathogenic micro-organisms in an individual or group pen (20) for young animals,
comprising a UV-C radiation source (30) that serves for said inactivation and is arranged on a stand (31) in an individual or group pen (20) or suspended from a ceiling in an individual or group pen (20) or attached to the ceiling,
comprising the steps of:
- removing a young animal from an individual pen (20) or young animals from a group pen (20),
- removing manure from the individual or group pen (20),
- cleaning the individual or group pen (20) and
- irradiating the floor surface (21), preferably the entire inner surface, of the individual or group pen (20) with UV-C radiation (32).

2. Method according to Claim 1, further comprising disinfecting the individual or group pen (20) prior to the irradiation with UV-C radiation (32).

3. Method according to Claim 1 or 2, wherein, when said device (100) is used, the latter further comprises a holder for a mixing container (60), wherein the holder is configured such that the UV-C radiation source (30) can be positioned, actively or passively, inside a mixing container (60) that is held by means of the holder.

## Revendications

1. Procédé d'inactivation de microorganismes pathogènes dans une étable individuelle ou collective (20) pour animaux juvéniles,
comprenant une source de rayonnement UV-C (30) destinée à ladite inactivation, laquelle est disposée sur un support (31) dans une étable individuelle ou collective (20) ou est suspendue à un plafond dans une étable individuelle ou collective (20) ou est fixée au plafond, comprenant les étapes suivantes :
- l'hébergement d'un animal juvénile dans une étable individuelle (20) ou d'animaux juvéniles dans une étable collective (20),
- l'élimination de la buée de l'étable individuelle ou collective (20),
- le nettoyage de l'étable individuelle ou collective (20), et
- l'irradiation de la surface du sol (21), de préférence la totalité de la surface intérieure, de l'étable individuelle ou collective (20) à l'aide d'un rayonnement UV-C (32).

2. Procédé selon la revendication 1, comprenant en outre la désinfection de l'étable individuelle ou collective (20) avant l'irradiation à l'aide d'un rayonnement UV-C (32).

3. Procédé selon la revendication 1 ou 2, dans lequel, lors de l'utilisation dudit dispositif (100), ledit dispositif comprend en outre un support pour un récipient de mélange (60), le support étant configuré de telle sorte que la source de rayonnement UV-C (30) peut être positionnée de manière active ou passive à l'intérieur d'un récipient de mélange (60) maintenu au moyen du support.
